# EUROPEAN PATENT APPLICATION

(11) **EP 4 708 304 A1**
(43) Date of publication of application: **11.03.2026**
(21) Application number: 25197730.2
(22) Date of filing: 22.08.2025
(51) Int. Cl.: G16C 20/30, G16C 20/40, G16C 20/70

(54) **CHEMICAL SIMILARITY SEARCH USING FINE-TUNED NEURAL NETWORK**

(30) Priority: 05.09.2024 US 202418825994
(71) Applicant: Microsoft Technology Licensing, LLC, Redmond, WA 98052 (US)
(72) Inventor: CHEN, Chi, Redmond, WA 98052 (US); LIU, Hongbin, Redmond, WA 98052 (US); ALMULLA, Yousif Waleed, Redmond, WA 98052 (US)
(74) Representative: CMS Cameron McKenna Nabarro Olswang LLP

(57) **Abstract**

Examples are disclosed that relate to forming embeddings comprising vector representations of chemical structures, and performing chemical similarity searches. One example provides a method of forming a vector database using a pre-trained neural network, the method comprising inputting (402) labeled training data into the pre-trained neural network configured to form embeddings of chemical structures, the labeled training data comprising structural information and a value of a property for each chemical object in a first set of chemical objects. The method further comprises fine-tuning (408) the pre-trained neural network and forming (414) the vector database by inputting a reference dataset into the fine-tuned neural network to generate embeddings of chemical structures, the reference dataset comprising structural information for each chemical object in a second set of chemical objects. Each embedding stored in the vector database comprises a vector representation of a chemical structure and embedded information for the property for the chemical structure.

## Description

### BACKGROUND

A chemical information database can be used to help search for chemicals that are structurally similar to a selected chemical of interest. Search methods often use structural comparisons that utilize rule-based algorithms.

### SUMMARY

This Summary is provided to introduce a selection of concepts in a simplified form that are further described below in the Detailed Description. This Summary is not intended to identify key features or essential features of the claimed subject matter, nor is it intended to be used to limit the scope of the claimed subject matter. Furthermore, the claimed subject matter is not limited to implementations that solve any or all disadvantages noted in any part of this disclosure.

Examples are disclosed that relate to forming embeddings of chemical structures, and performing chemical similarity searches based on embeddings. Embeddings are high-dimensional vector representations of chemical structures. One example provides a method of forming a vector database using a pre-trained neural network, the method comprising inputting labeled training data into the pre-trained neural network configured to form embeddings of chemical structures, the labeled training data comprising structural information and a value of a property for each chemical object in a first set of chemical objects, and fine-tuning the pre-trained neural network using the labeled training data, thereby forming a fine-tuned neural network. The method further comprises forming the vector database by inputting a reference dataset into the fine-tuned neural network to generate embeddings of chemical structures, the reference dataset comprising structural information for each chemical object in a second set of chemical objects, each embedding comprising a vector representation of a chemical structure and embedded information for the property for the chemical structure. The method further comprises storing the embeddings with metadata in the vector database.

Another example provides a method enacted on a computing system, the method comprising inputting labeled training data into a pre-trained neural network configured to form embeddings of chemical structures, the labeled training data comprising structural information and a value for a property for each chemical object in a set of chemical objects, and fine-tuning the pre-trained neural network using the labeled training data, thereby forming a fine-tuned neural network. The method further comprises forming a vector database by using the fine-tuned neural network to generate embeddings of chemical structures of a reference dataset, each embedding comprising a vector representation of a chemical structure and embedded information for the property for the chemical structure, and storing the embeddings with metadata in the vector database. The method further comprises receiving a query comprising chemical structure information for a target chemical object and, based on the query, inputting the chemical structure information into the fine-tuned neural network. The method further comprises receiving an embedding for the target chemical object from the fine-tuned neural network, the embedding for the target chemical object comprising a vector representation of the target chemical object. The method further comprises, based at least on a similarity score between the embedding for the target chemical object and each of one or more embeddings stored in the vector database, retrieving query results from the vector database, the query results comprising a set of embeddings and metadata for a corresponding set of chemical objects, and outputting the query results.

Another example provides a computing system implementing a neural network configured to produce embeddings of chemical structures, the computing system comprising a logic subsystem, and a storage subsystem comprising instructions executable by the logic subsystem to input labeled training data into a pre-trained neural network configured to form embeddings of chemical structures, the labeled training data comprising structural information and a value for a property for each chemical object in a first set of chemical objects. The instructions are further executable to train the neural network to predict the property, thereby forming a fine-tuned neural network, input a reference dataset into the fine-tuned neural network to generate embeddings of chemical structures, the reference dataset comprising structural information for a second set of chemical objects, each embedding comprising a vector representation of a chemical structure and embedded information for the property for the chemical structure, and store the embeddings with metadata in a vector database.

Examples are disclosed that relate to forming embeddings comprising vector representations of chemical structures, and performing chemical similarity searches. One example provides a method of forming a vector database using a pre-trained neural network, the method comprising inputting labeled training data into the pre-trained neural network configured to form embeddings of chemical structures, the labeled training data comprising structural information and a value of a property for each chemical object in a first set of chemical objects. The method further comprises fine-tuning the pre-trained neural network and forming the vector database by inputting a reference dataset into the fine-tuned neural network to generate embeddings of chemical structures, the reference dataset comprising structural information for each chemical object in a second set of chemical objects. Each embedding stored in the vector database comprises a vector representation of a chemical structure and embedded information for the property for the chemical structure.

### BRIEF DESCRIPTION OF THE DRAWINGS

FIG. 1 schematically shows an example of a computing architecture for implementing a searchable vector database.
FIG. 2 shows a flow diagram for an example method of processing a chemical search query.
FIG. 3 shows a flow diagram for an example method of training a neural network to learn embeddings and forming a vector database comprising embeddings.
FIG. 4 shows a flow diagram for an example method of fine-tuning a pre-trained neural network to form embeddings that contain embedded property information for chemical structures.
FIG. 5 shows a block diagram of an example computing system.

### DETAILED DESCRIPTION

In the field of cheminformatics and materials science, it can be challenging to compare chemicals, or to identify materials with a desired property. Chemical information databases can be used to help search for chemicals that are structurally similar to a target chemical. However, search methods often use simplistic structural comparisons that utilize rule-based algorithms or fingerprint methods. Such structural comparisons can fail to account for nuanced similarities and/or functional relationships. Search algorithms can include additional rules to attempt to capture a greater number of functional relationships. However, more complicated algorithms can be slow.

Accordingly, examples are disclosed that relate to using deep learning models to generate embeddings for chemical objects. Deep learning models can include, for example, graph neural networks or transformers. Examples of chemical objects that can be represented via embeddings include molecules, materials, and/or crystal structures. After generating embeddings, the embeddings can be used to form a vector database for use in a chemical similarity search. Briefly, a neural network is trained using training data comprising chemical structure data. Training can be supervised or unsupervised, as described in more detail below. During training, one or more embedding layers learn to generate latent representations, or embeddings, of chemical structures. After training, the neural network can be used to generate embeddings of chemical objects.

The trained neural network further can be used in a fine-tuning process to incorporate chemical and/or physical property information into the embeddings. This allows the resulting vector database to be used for a chemical similarity search that combines chemical structure and property information. Briefly, after training the neural network using chemical structure data, the pre-trained neural network is fine-tuned using labeled training data comprising property information for a chemical property (e.g., electronegativity) or physical property (e.g., boiling point). After fine-tuning, the neural network can be used to generate embeddings of chemical objects. The embeddings formed with the fine-tuned neural network comprise embedded information related to the property, in addition to the embedded structural information. Compared to training from scratch, fine-tuning can leverage prior understanding of chemistry captured by the pre-trained model. This helps the fine-tuned model represent structural and functional characteristics with greater accuracy than a model trained from scratch. Further, the fine-tuning process can leverage the large training set used for the pre-trained neural network and fine-tunes it using a relatively smaller dataset of labeled training data. As such, a pre-trained neural network can be fine-tuned in a robust and cost-efficient manner.

Embeddings can capture intricate properties and relationships of chemical structures in a multidimensional space. To generate an embedding, a chemical structure is input into the trained neural network, and the embedding for the chemical structure is output by the embedding layer. The embedding comprises a high-dimensional vector representation of the chemical structure, wherein the vector representation is formed from coefficients output by the embedding layer of the trained neural network. The generated embeddings can be stored in a vector database. Embeddings are stored with metadata comprising information identifying the chemical objects corresponding to the embeddings. The vector database can then be used for performing a chemical similarity search. As chemical structures are represented by high dimensional vectors, chemical similarity between chemical objects can be measured using a Euclidean distance, a Manhattan distance, a dot product, or a cosine similarity, as examples. Further, embeddings from fine-tuned neural networks comprise vector representations that encapsulate structural and functional characteristics of the molecules. By comparing these embeddings to those of a target molecule, the system can efficiently identify chemical alternatives that share desired structure and/or properties.

To query the vector database, chemical structure information for a target chemical object is input into the trained neural network. The chemical structure information can include, e.g., cartesian coordinates of atoms in a chemical object and/or bonding information for a chemical object. Examples of inputs of chemical structure information include text strings (e.g., a chemical name or a simplified molecular-input line-entry system (SMILES) string) and 3-dimensional (3D) structural data (e.g., XYZ format, crystallographic information file (CIF) format, MOL format, Protein Data Bank (PDB) format, etc.). The trained neural network generates an embedding comprising a vector representation of the target chemical object. The embedding is used to query the vector database to identify other chemical objects that are structurally similar to the target chemical object. As described in more detail below, the structural similarity of two chemical objects can be quantified based on the respective embeddings for the chemical objects. In this manner, one or more chemical objects that are structurally similar to the target chemical object can be identified and output to a user. In examples where a fine-tuned neural network is used, the embedding comprises embedded structural information and embedded property information. This allows for a vector-based search to identify chemical objects that are structurally similar and have similar property values to the target chemical object.

A vector database can offer improvements over traditional cheminformatics databases that are not optimized for high-dimensional data produced by deep-learning models. By using a vector database rather than a rule-based search method, querying can be more efficient than other methods. Additionally, the vector database can be configured for storing embeddings and high-efficiency querying of embeddings. The computational efficiency of the method also can surpass traditional algorithms (e.g., rule-based algorithms or fingerprint methods), as the embedding process condenses complex information into a manageable form. By using a high-dimensional vector representation of complex information, the system facilitates fast, scalable searches within the vector database. In some examples, 100 million entries can be searched in less than 0.1 seconds. Further, by using deep learning for embeddings, the system can capture complex molecular characteristics that other methods can overlook. This helps provide chemical similarity searches with greater accuracy and more meaningful results than other search methods. This can be useful in various applications, such as drug discovery, material design, and environmental studies.

Additionally, the use of fine-tuned neural network-based embeddings with embedded property information allows for a more nuanced representation of chemical objects, capturing structural intricacies and functional characteristics within a single vector. This dual representation is a significant advancement over methods that consider structural or property data in isolation. Additionally, by extracting embeddings from advanced neural network architectures, for example, fine-tuned Graph Neural Networks (GNNs), the system can discern subtle chemical and physical relationships that are often overlooked by conventional algorithms. This leads to more accurate and relevant search results, such as identifying viable alternatives for environmentally or health-sensitive chemicals. This efficiency, combined with the scalability, provides a valuable tool for rapid and precise chemical exploration and innovation.

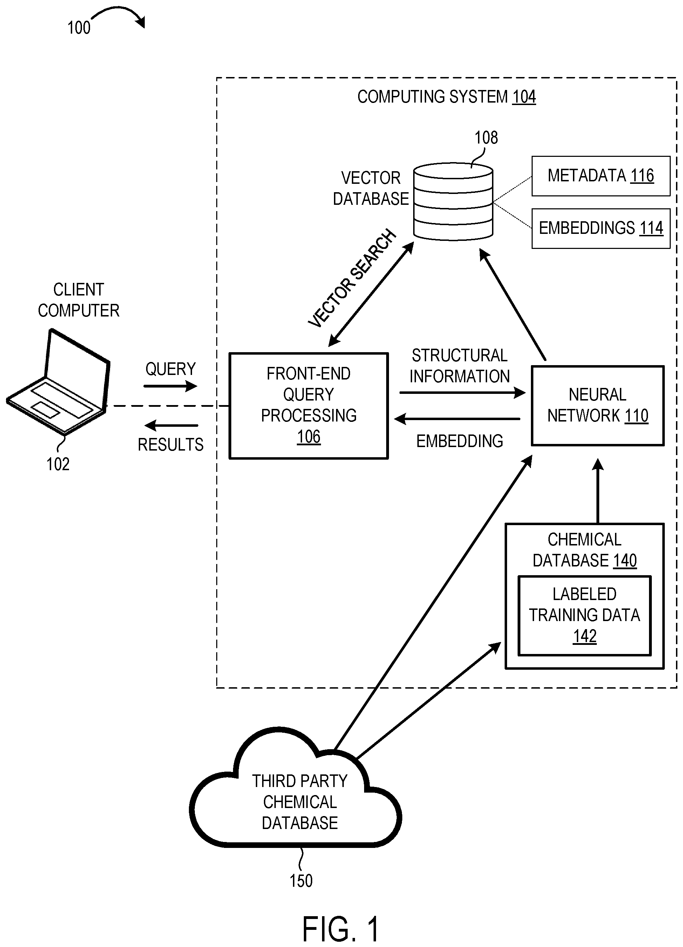

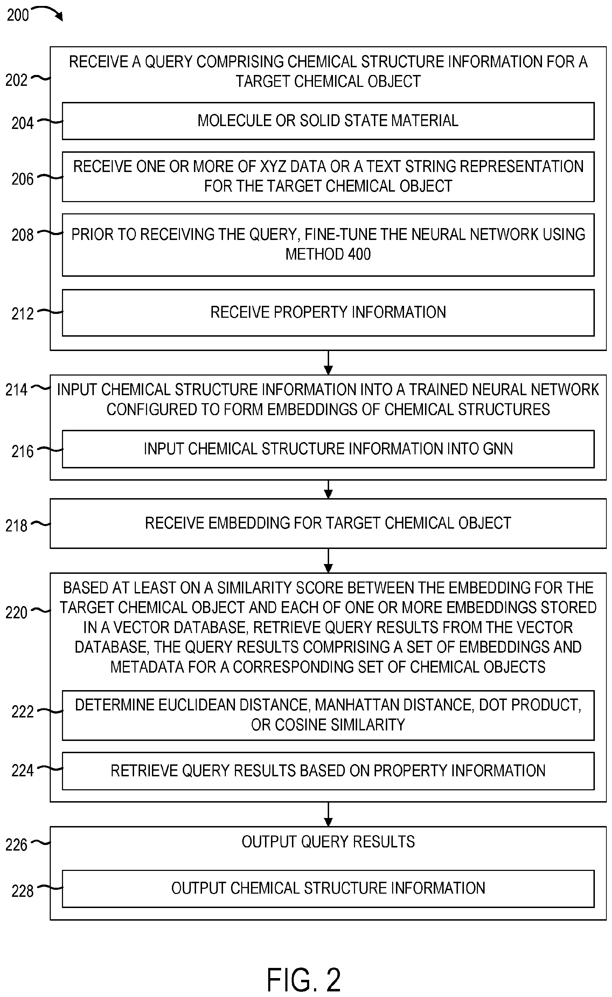

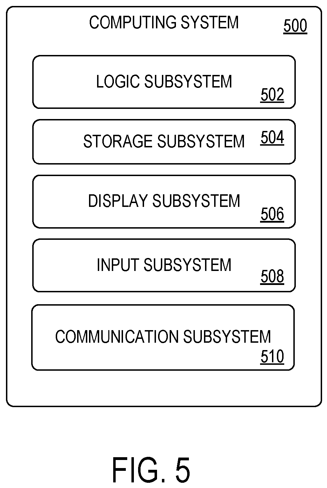

FIG. 1 schematically shows an example of a computing architecture 100 for processing a chemical search query. As illustrated in FIG. 1, a client computer 102 can submit a query to a computing system 104. Computing system 104 comprises one or more processors configured to process queries and perform various other functions. An example method of processing queries is discussed below with regard to FIG. 2. Computing system 104 can represent a data center in some examples. Examples of computing systems are described in more detail below with regard to FIG. 5.

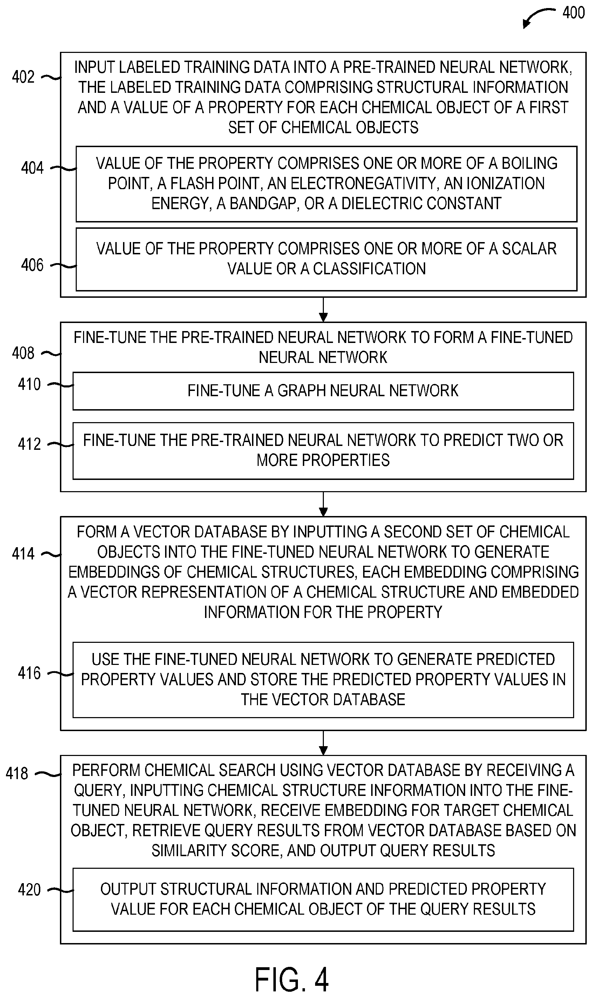

Computing system 104 comprises a front-end module 106 for query processing. Computing system 104 further comprises a storage system storing data for a vector database 108, one or more neural networks 110, and training data 112. Vector database 108 comprises a plurality of embeddings 114 for a respective plurality of chemical objects, including molecules and/or materials (e.g., crystal structures or other representation of solids). Each embedding comprises a vector representation of the structure of a chemical object. The vector database further comprises metadata 116. Metadata 116 can comprise any suitable information for chemical objects corresponding to embeddings 114. In some examples, metadata 116 can include, for each embedding, a chemical object identification (ID) corresponding to the embedding. Examples of metadata include chemical names, chemical formulas, and ID numbers. In some examples, vector database 108 can optionally comprise property data for one or more chemical objects corresponding to embeddings 114. In some examples, the property data comprises an energy for a chemical object, such as a formation energy. Additional examples of properties include a boiling point, a flash point, an ionization energy, a bandgap, and a dielectric constant. Further examples include classifications, such as metal vs. non-metal or conducting vs. insulating. In various examples, the property data can comprise experimental properties and/or predicted properties. However, property data may not exist for some chemical objects. In some examples, predicted property data can be generated during a fine-tuning process, as discussed in more detail below with regard to FIG. 4.

Neural network 110 is configured to generate embeddings for chemical objects. Examples of neural networks include transformers, graph-transformers, convolutional neural networks (CNNs), and/or GNNs. GNNs are trained to perform inference on data described by a mathematical graph. Graphs can be a suitable choice for representing a chemical object, such as a molecule or solid state material (e.g. a unit cell of a crystal), where nodes represent atoms and edges represent bonds. Examples of GNNs suitable for generating embeddings include GNNs trained using the Graphormer deep learning package (Ying, Chengxuan, et al. "Do transformers really perform badly for graph representation?." Advances in neural information processing systems 34 (2021): 28877-28888.). Any suitable configuration can be used for neural network 110. In some examples, neural network 110 comprises a GNN configured with ≥ 6 layers. In some examples, neural network 110 comprises ≥ 80 hidden dimensions. In some more specific examples, neural network 110 comprises 12 layers, 32 attention heads, and 24 hidden dimensions for each attention head. In other examples, any other suitable configuration can be used.

Neural network 110 can be trained using any suitable training data. Computing system 104 optionally comprises a chemical database 140 that can be used to provide training data. Chemical database 140 can comprise one or more sets of chemical objects. In some examples, chemical database 140 comprises labeled training data 142 comprising structural information and property information for each chemical object of a set of chemical objects. The property information can comprise values for one or more properties (e.g., boiling point). Labeled training data 142 can be used in a fine-tuning process as described below. Additionally or alternatively, a third-party chemical database 150 can be used to provide training data for training neural network 110. One example of a publicly accessible third-party chemical database is PubChem, available at pubchem.ncbi.nlm.nih.gov (Kim S, Chen J, Cheng T, et al. PubChem 2023 update. Nucleic Acids Res. 2023;51(D1):D1373-D1380.).

Neural network 110 can be trained using supervised training or unsupervised training. In supervised training, labeled training data is input into neural network 110 and the neural network is trained to predict a property, such as an energy. In some examples, a computationally inexpensive energy calculation can be used to predict energies for use in the supervised training process. In other examples, any other suitable predicted property can be used. In further example, empirical data can be used.

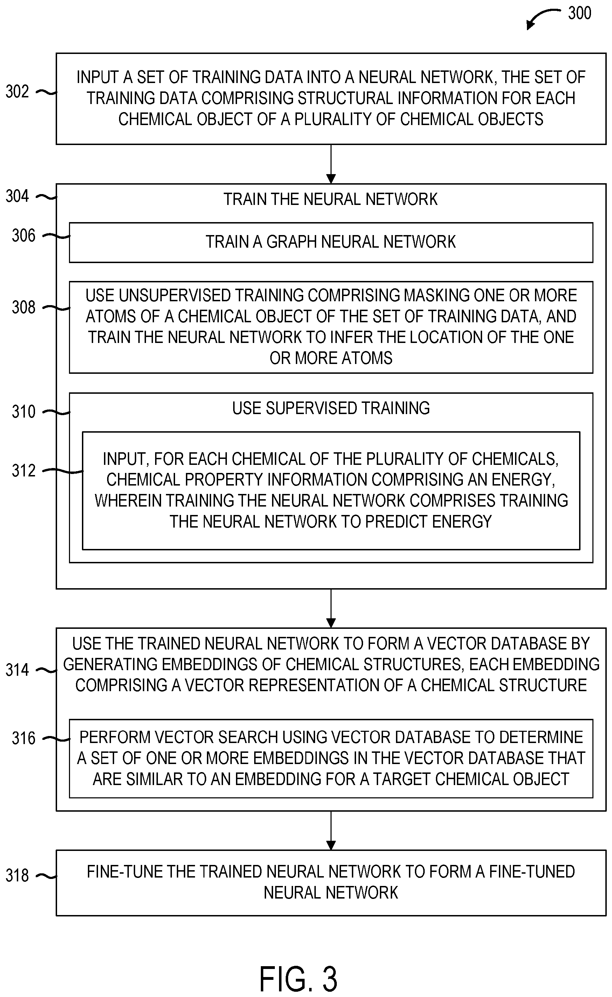

Alternatively, neural network 110 can be trained using unsupervised training. In unsupervised training, the neural network can be trained to predict structural information of a chemical object. For example, one or more atoms of a chemical object can be masked, and the neural network learns to predict a type and/or location of the masked atom in the chemical object. Examples for training neural network 110 are discussed below with regard to FIG. 3.

After training, neural network 110 can be used to generate embeddings based on chemical structures. As discussed above, an embedding comprises a vector representation of a chemical structure. In some examples, the length of the vector corresponds to the number of hidden dimensions in the neural network. Embeddings can be used to form vector database 108. For example, a plurality of chemical objects from chemical database 140 and/or third-party chemical database 150 can be input into neural network 110 to generate embeddings. In some examples, a chemical database can be supplied by a user (e.g., from client computer 102). The generated embeddings are stored in vector database 108 as embeddings 114. Each embedding can be stored with metadata 116, such as a chemical object ID corresponding to the embedding.

Neural network 110 can be further trained using additional training data comprising new chemical objects. In contrast to static rule-based algorithms, computing system 104 can continuously learn and update neural network 110 to understand additional chemical structures. After the further training, neural network 110 can be used to update embeddings 114. This dynamic learning aspect allows vector database 108 to adapt to new data and discoveries.

Additionally, after initial training (pre-training) using structural data, neural network 110 can be fine-tuned. A fine-tuning process can include inputting labeled training data into a pre-trained version of training neural network 110 to predict a chemical or physical property. Examples of properties include a boiling point, a flash point, an ionization energy, a bandgap, a dielectric constant, and classifications, such as metal vs. non-metal or conducting vs. insulating. After fine-tuning, neural network 110 can be used to generate embeddings for storage in vector database 108. As mentioned above, embeddings generated by a fine-tuned version of neural network 110 comprise embedded structural information and embedded property information. This allows for a vector-based search to identify chemical objects that are structurally similar and have similar property values to the target chemical object. As property data may not exist for some of the chemical objects stored in the vector database, the vector-based search can help provide chemical similarity search functionality that is not feasible using other search methods. In some examples, fine-tuning can be performed using user data. For example, client computer 102 can transmit labeled training data to computing system 104 to be used in the fine-tuning process. In some such examples, the fine-tuning process can be used to form a user version of vector database 108. This can allow a user to form a customized vector database without affecting a public version of vector database 108. Fine-tuning is discussed in more detail below with regard to FIG. 4.

Further, neural network 110 can be used to generate an embedding that can be used in a chemical search query. For example, in response to a query from client computer 102 received at front-end module 106, computing system 104 inputs structural information for a target chemical object into neural network 110. Neural network 110 generates an embedding comprising a vector representation of the target chemical object. The embedding can be output to front-end module 106. Computing system 104 can then perform a vector search of vector database 108 to retrieve embeddings similar to the embedding for the target chemical object. The vector search can employ a similarity score, for example. Any suitable similarity score can be used, such as a Euclidean distance, a Manhattan distance, a dot product, or a cosine similarity. A relatively high degree of similarity between two embeddings indicates a relatively high degree of topological similarity between the two chemical objects corresponding to the embeddings. Thus, based on the embedding of the target chemical object and a similarity score metric, the vector search can retrieve a set of K similar chemical objects from the vector database. In examples where a fine-tuned neural network is used to generate embeddings, the embedding captures structural and functional characteristics within a single vector. In some examples, a chemical search query can include explicit property information and the query results can be weighted and/or filtered based on the property information specified in the query.

Embeddings can capture complex molecular characteristics and intricate relationships between chemicals in a multidimensional space. In this manner, embeddings can be more nuanced than other solutions that utilize rule-based algorithms or fingerprint methods for structural comparisons between different chemical structures. As such, a vector search of vector database 108 can provide a chemical similarity search with greater accuracy and more meaningful results than other search methods.

FIG. 2 shows a flow diagram for an example method 200 for processing a chemical search query using a vector database (e.g., vector database 108) and a neural network (e.g., neural network 110). Computing architecture 100 is an example of a computing architecture for processing chemical search queries using method 200.

Method 200 comprises, at 202, receiving a query comprising chemical structure information for a target chemical object. In some examples, at 204, the chemical structure information for the target chemical object comprises structural information for a molecule or structural information for a solid state material. The query can comprise any suitable chemical structure information. In some examples, at 206, method 200 comprises receiving one or more of XYZ data for the target chemical object, or a text string representation of the target chemical object. XYZ data can comprise cartesian coordinates of atoms in a chemical object using any suitable format. Examples of XYZ data includes chemical structure information in an XYZ format, CIF format, MOL format, and PDB format. A text string representation can comprise any suitable information identifying a chemical object or describing bonding within a chemical object. Examples of text string representations for a chemical object include a chemical name, a chemical formula, and a SMILES string.

In some examples, at 208, method 200 comprises fine-tuning the neural network prior to receiving the query at 202. For example, fine-tuning the neural network can be performed using method 400 described below with regard to FIG. 4. In some examples, at 212, receiving the query comprises receiving property information. Property information can be used, e.g., to filter query results or refine a chemical similarity search based on predicted property values.

Method 200 further comprises, at 214, inputting the chemical structure information into a trained neural network configured to form embeddings of chemical structures. Any suitable neural network can be used. Neural network 110 is an example of a neural network that can be used at step 214. In some examples, at 216, the chemical structure information is input into a GNN.

Method 200 further comprises, at 218, receiving an embedding for the target chemical object from the trained neural network, the embedding comprising a vector representation of the target chemical object.

Continuing, at 220, method 200 further comprises, based at least on a similarity score between the embedding for the target chemical object and each of one or more embeddings stored in a vector database, retrieving query results from the vector database. The query results comprise a set of embeddings and metadata for a corresponding set of chemical objects. The metadata can comprise, e.g., chemical object IDs for the corresponding set of chemical objects. Any suitable method can be used to determine a similarity score at step 220. In some examples, at 222, retrieving the query results comprises determining one or more of a Euclidean distance, a Manhattan distance, a dot product, or a cosine similarity for the embedding for the target chemical object and an embedding stored in the vector database. In some examples, at 224, retrieving query results from the vector database is further based on property information and the predicted values for the property for corresponding chemical objects of the query results. Additionally or alternatively, when fine-tuning is performed at step 208, functional characteristics related to the property used in the fine-tuning process can be captured by the embedding. As such, structural similarity and property similarity can be implicitly included in the vector search.

Method 200 further comprises, at 226, outputting the query results. Referring to FIG. 1, computing system 104 can output query results to client computer 102. Any suitable information pertaining to the query results can be output, such as a chemical name or a chemical formula for each chemical object in the query results. In some examples, at 228, method 200 comprises, for each chemical object in the one or more chemical objects of the query results, outputting structural information for the chemical object. In some examples, method 200 comprises outputting property information.

FIG. 3 shows a flow diagram for an example method 300 of training a neural network (e.g., neural network 110). At 302, method 300 comprises inputting a set of training data into a neural network, the set of training data comprising structural information for each chemical object of a plurality of chemical objects. In some examples, the set of training data comprises labeled training data.

At 304, method 300 further comprises training the neural network. In some examples, at 306, training the neural network comprises training a graph neural network (GNN). As discussed above, a graph can be a suitable choice for representing a chemical object. In some examples, at 308, training the neural network comprises using unsupervised training, wherein the unsupervised training comprises masking one or more atoms of a chemical object of the set of training data, and training the neural network to predict the location of the one or more atoms. In some examples, at 310, training the neural network comprises using supervised training. Supervised training uses labeled training data. In some such examples, at 312, the method comprises inputting, for each chemical object of the plurality of chemical objects, chemical property information comprising an energy, and wherein training the neural network comprises training the neural network to predict energy.

Continuing, at 314, method 300 further comprises using the trained neural network to form a vector database by generating embeddings of chemical structures, each embedding comprising a vector representation of a chemical structure. The embeddings are stored in the vector database with metadata (e.g., IDs for the chemical objects associated with the embeddings). In some examples, at 316, method 300 further comprises performing a vector search using the vector database to determine a set of one or more embeddings in the vector database that are similar to an embedding for a target chemical object. In some examples, at 318, method 300 comprises fine-tuning the trained neural network to form a fine-tuned neural network.

FIG. 4 shows a flow diagram of an example method 400 for fine-tuning a neural network. At 402, method 400 comprises inputting labeled training data (e.g., labeled training data 142) into a pre-trained neural network. In some examples, method 400 can comprise performing method 300 to form the pre-trained neural network. The labeled training data comprises structural information and a value of a property for each chemical object of a first set of chemical objects. In some examples, the labeled training data can comprise two or more values for a corresponding two or more properties. In some examples, at 404, the value of the property comprises one or more of a boiling point, a flash point, an ionization energy, a bandgap, or a dielectric constant. In some examples, at 406, the value of the property comprises one or more of a scalar value or a classification. Examples of scalar values include those listed at step 404. Examples of classifications include metal, non-metal, conducting, or insulating.

Method 400 further comprises, at 408, fine-tuning the pre-trained neural network to form a fine-tuned neural network. In some examples, at 410, step 408 comprises fine-tuning a GNN. In some examples, at 412, step 408 comprises fine-tuning the pre-trained neural network to predict two or more properties.

Continuing, method 400 further comprises, at 414, forming a vector database (e.g., vector database 108) by inputting a second set of chemical objects (e.g., a reference dataset from chemical database 140 or third-party database 150) into the fine-tuned neural network to generate embeddings of chemical structures. Each embedding comprises a vector representation of a chemical structure and embedded information for the property. In some examples, at 416, the method comprises using the fine-tuned neural network to generate predicted property values for the property, and storing the predicted property values with corresponding vectors in the vector database.

In some examples, at 418, method 400 optionally comprises performing a chemical search using the vector database formed at 414. The chemical search comprises receiving a query comprising chemical structure information for a target chemical object. The chemical search further comprises, based on the query, inputting the chemical structure information for the target chemical object into the fine-tuned neural network. The chemical search further comprises receiving an embedding for the target chemical object from the fine-tuned neural network, the embedding comprising a vector representation of the target chemical object. The chemical search further comprises, based at least on a similarity score between the embedding for the target chemical object and each of one or more embeddings stored in the vector database, retrieving query results from the vector database, the query results comprising a set of embeddings and metadata (e.g., metadata 116) for a corresponding set of chemical objects. As mentioned above, the similarity score can be determined using any suitable method, such as by determining a Euclidean distance, a Manhattan distance, a dot product, and/or a cosine similarity. The chemical search further comprises outputting the query results. In some examples, at 420, method 400 comprises outputting structural information and a predicted property value (e.g., from property data generated at step 416) for each chemical object of the query results.

Thus, the disclosed examples provide for generating vector databases using a neural network and performing a chemical similarity search using the vector database. After fine-tuning a neural network for a selected property, the embeddings generated from the fine-tuned neural network comprise vector representations that encapsulate structural and functional characteristics of the molecules. A vector search utilizing such embeddings can efficiently identify chemical alternatives that share structure and/or properties with a target chemical object. By using deep learning for embeddings, the disclosed examples can capture complex molecular characteristics that other methods might overlook, leading to more accurate and meaningful similarity searches. Further, the trained neural network can form embeddings for a wide range of chemical structures. This makes it applicable in various fields, from drug discovery to materials engineering.

In some embodiments, the methods and processes described herein may be tied to a computing system of one or more computing devices. In particular, such methods and processes may be implemented as a computer-application program or service, an application-programming interface (API), a library, and/or other computer-program product.

FIG. 5 schematically shows a non-limiting embodiment of a computing system 500 that can enact one or more of the methods and processes described above. Computing system 500 is shown in simplified form. Computing system 500 may take the form of one or more personal computers, server computers, tablet computers, home-entertainment computers, network computing devices, gaming devices, mobile computing devices, mobile communication devices (e.g., smart phone), and/or other computing devices. Computing system 104 is an example of computing system 500.

Computing system 500 includes a logic subsystem 502 and a storage subsystem 504. Computing system 500 may optionally include a display subsystem 506, input subsystem 508, communication subsystem 510, and/or other components not shown in FIG. 5.

Logic subsystem 502 includes one or more physical devices configured to execute instructions. For example, the logic subsystem may be configured to execute instructions that are part of one or more applications, services, programs, routines, libraries, objects, components, data structures, or other logical constructs. Such instructions may be implemented to perform a task, implement a data type, transform the state of one or more components, achieve a technical effect, or otherwise arrive at a desired result.

The logic subsystem may include one or more processors configured to execute software instructions. Additionally or alternatively, the logic subsystem may include one or more hardware or firmware logic subsystems configured to execute hardware or firmware instructions. Processors of the logic subsystem may be single-core or multi-core, and the instructions executed thereon may be configured for sequential, parallel, and/or distributed processing. Individual components of the logic subsystem optionally may be distributed among two or more separate devices, which may be remotely located and/or configured for coordinated processing. Aspects of the logic subsystem may be virtualized and executed by remotely accessible, networked computing devices configured in a cloud-computing configuration.

Storage subsystem 504 includes one or more physical devices configured to hold instructions executable by the logic subsystem to implement the methods and processes described herein. When such methods and processes are implemented, the state of storage subsystem 504 may be transformed-e.g., to hold different data.

Storage subsystem 504 may include removable and/or built-in devices. Storage subsystem 504 may include optical memory (e.g., CD, DVD, HD-DVD, Blu-Ray Disc, etc.), semiconductor memory (e.g., RAM, EPROM, EEPROM, etc.), and/or magnetic memory (e.g., hard-disk drive, floppy-disk drive, tape drive, MRAM, etc.), among others. Storage subsystem 504 may include volatile, nonvolatile, dynamic, static, read/write, read-only, random-access, sequential-access, location-addressable, file-addressable, and/or content-addressable devices.

It will be appreciated that storage subsystem 504 includes one or more physical devices. However, aspects of the instructions described herein alternatively may be propagated by a communication medium (e.g., an electromagnetic signal, an optical signal, etc.) that is not held by a physical device for a finite duration.

Aspects of logic subsystem 502 and storage subsystem 504 may be integrated together into one or more hardware-logic components. Such hardware-logic components may include field-programmable gate arrays (FPGAs), program- and application-specific integrated circuits (PASIC / ASICs), program- and application-specific standard products (PSSP / ASSPs), system-on-a-chip (SOC), and complex programmable logic devices (CPLDs), for example.

The terms "module," "program," and "engine" may be used to describe an aspect of computing system 500 implemented to perform a particular function. In some cases, a module, program, or engine may be instantiated via logic subsystem 502 executing instructions held by storage subsystem 504. It will be understood that different modules, programs, and/or engines may be instantiated from the same application, service, code block, object, library, routine, API, function, etc. Likewise, the same module, program, and/or engine may be instantiated by different applications, services, code blocks, objects, routines, APIs, functions, etc. The terms "module," "program," and "engine" may encompass individual or groups of executable files, data files, libraries, drivers, scripts, database records, etc.

It will be appreciated that a "service", as used herein, is an application program executable across multiple user sessions. A service may be available to one or more system components, programs, and/or other services. In some implementations, a service may run on one or more server-computing devices.

When included, display subsystem 506 may be used to present a visual representation of data held by storage subsystem 504. This visual representation may take the form of a graphical user interface (GUI). As the herein described methods and processes change the data held by the storage subsystem, and thus transform the state of the storage subsystem, the state of display subsystem 506 may likewise be transformed to visually represent changes in the underlying data. Display subsystem 506 may include one or more display devices utilizing virtually any type of technology. Such display devices may be combined with logic subsystem 502 and/or storage subsystem 504 in a shared enclosure, or such display devices may be peripheral display devices.

When included, input subsystem 508 may comprise or interface with one or more user-input devices such as a keyboard, mouse, touch screen, or game controller. In some embodiments, the input subsystem may comprise or interface with selected natural user input (NUI) componentry. Such componentry may be integrated or peripheral, and the transduction and/or processing of input actions may be handled on- or off-board. Example NUI componentry may include a microphone for speech and/or voice recognition; an infrared, color, stereoscopic, and/or depth camera for machine vision and/or gesture recognition.

When included, communication subsystem 510 may be configured to communicatively couple computing system 500 with one or more other computing devices. Communication subsystem 510 may include wired and/or wireless communication devices compatible with one or more different communication protocols. As non-limiting examples, the communication subsystem may be configured for communication via a wireless telephone network, or a wired or wireless local- or wide-area network. In some embodiments, the communication subsystem may allow computing system 500 to send and/or receive messages to and/or from other devices via a network such as the Internet.

Clause A. A method of forming a vector database using a fine-tuned neural network, the method comprising: inputting labeled training data into a pre-trained neural network configured to form embeddings of chemical structures, the labeled training data comprising structural information and a value of a property for each chemical object in a first set of chemical objects; fine-tuning the pre-trained neural network using the labeled training data, thereby forming the fine-tuned neural network; and forming the vector database by inputting a reference dataset into the fine-tuned neural network to generate embeddings of chemical structures, the reference dataset comprising structural information for each chemical object in a second set of chemical objects, each embedding comprising a vector representation of a chemical structure and embedded information for the property for the chemical structure, and storing the embeddings with metadata in the vector database.

Clause B. The method of clause A, further comprising, prior to inputting the labeled training data, inputting a set of pre-training data into a neural network, the set of pre-training data comprising structural information for each chemical object of a third set of chemical objects; and training the neural network to form the pre-trained neural network.

Clause C. The method of either of clauses A or B, further comprising: receiving a query comprising chemical structure information for a target chemical object; based on the query, inputting the chemical structure information for the target chemical object into the fine-tuned neural network; receiving an embedding for the target chemical object from the fine-tuned neural network, the embedding comprising a vector representation of the target chemical object; based at least on a similarity score between the embedding for the target chemical object and each of one or more embeddings stored in the vector database, retrieving query results from the vector database, the query results comprising a set of embeddings and metadata for a corresponding set of chemical objects; and outputting the query results.

Clause D. The method of clause C, wherein outputting the query results comprises, for each chemical object in the corresponding set of chemical objects of the query results, outputting structural information and a predicted property value for the chemical object.

Clause E. The method of either of clause C or D, wherein retrieving the query results from the vector database comprises determining one or more of a Euclidean distance, a Manhattan distance, a dot product, or a cosine similarity for the embedding for the target chemical object and an embedding stored in the vector database.

Clause F. The method of any of clauses A to E, wherein the value for the property comprises one or more of a scalar value or a classification for the property for each chemical object in the first set of chemical objects.

Clause G. The method of any of clauses A to F, wherein the labeled training data comprises values for two of more properties for each chemical object in the first set of chemical objects.

Clause H. The method of any of clauses A to G, wherein fine-tuning the neural network comprises fine-tuning a graph neural network (GNN).

Clause I. A method enacted on a computing system, the method comprising: inputting labeled training data into a pre-trained neural network configured to form embeddings of chemical structures, the labeled training data comprising structural information and a value for a property for each chemical object in a set of chemical objects; fine-tuning the pre-trained neural network using the labeled training data, thereby forming a fine-tuned neural network; forming a vector database by using the fine-tuned neural network to generate embeddings of chemical structures of a reference dataset, each embedding comprising a vector representation of a chemical structure and embedded information for the property for the chemical structure, and storing the embeddings with metadata in the vector database; receiving a query comprising chemical structure information for a target chemical object; based on the query, inputting the chemical structure information into the fine-tuned neural network; receiving an embedding for the target chemical object from the fine-tuned neural network, the embedding for the target chemical object comprising a vector representation of the target chemical object; based at least on a similarity score between the embedding for the target chemical object and each of one or more embeddings stored in the vector database, retrieving query results from the vector database, the query results comprising a set of embeddings and metadata for a corresponding set of chemical objects; and outputting the query results.

Clause J. The method of clause I, further comprising, prior to inputting the labeled training data, inputting a set of pre-training data into a neural network, the set of pre-training data comprising structural information for each chemical object of the dataset; and training the neural network to form the pre-trained neural network.

Clause K. The method of either of clauses I or J, wherein the value for the property comprises one or more of a scalar value or a classification for the property for each chemical object in the set of chemical objects.

Clause L. The method of any of clauses I to K, wherein the labeled training data comprises values for two of more properties for each chemical object in the set of chemical objects.

Clause M. The method of any of clauses I to L, further comprising, for each chemical object in the corresponding set of chemical objects of the query results, outputting structural information and a predicted property value for the chemical object.

Clause N. The method of any of clauses I to M, wherein retrieving the query results from the vector database comprises determining one or more of a Euclidean distance, a Manhattan distance, a dot product, or a cosine similarity for the embedding for the target chemical object and an embedding stored in the vector database.

Clause O. The method of any of clauses I to N, wherein fine-tuning the neural network comprises fine-tuning a graph neural network (GNN).

Clause P. A computing system implementing a neural network configured to produce embeddings of chemical structures, the computing system comprising: a logic subsystem; and a storage subsystem comprising instructions executable by the logic subsystem to input labeled training data into a pre-trained neural network configured to form embeddings of chemical structures, the labeled training data comprising structural information and a value for a property for each chemical object in a first set of chemical objects, train the neural network to predict the property, thereby forming a fine-tuned neural network, input a reference dataset into the fine-tuned neural network to generate embeddings of chemical structures, the reference dataset comprising structural information for a second set of chemical objects, each embedding comprising a vector representation of a chemical structure and embedded information for the property for the chemical structure, and store the embeddings with metadata in a vector database.

Clause Q. The computing system of clause P, wherein the instructions are further executable to: receive a query comprising chemical structure information for a target chemical object, based on the query, input the chemical structure information into the fine-tuned neural network, receive, from the fine-tuned neural network, an embedding comprising a vector representation of the target chemical object, based at least on a similarity score between the embedding for the target chemical object and each of one or more embeddings stored in the vector database, retrieve query results from the vector database, the query results comprising a set of embeddings and metadata for a corresponding set of chemical objects, and output the query results.

Clause R. The computing system of clause Q, wherein the instructions executable to output the query results are executable to output structural information and predicted property values for each chemical object in the corresponding set of chemical objects.

Clause S. The computing system of any of clauses P to R, wherein the labeled training data comprises values for two or more properties for each chemical object in the first set of chemical objects, and the instructions are executable to train the neural network to predict the two or more properties.

Clause T. The computing system of any of clauses P to S, wherein the instructions executable to train the neural network to predict the property comprise instructions executable to train a graph neural network to predict one or more of a boiling point, a flash point, an electronegativity, an ionization energy, a bandgap, or a dielectric constant.

It will be understood that the configurations and/or approaches described herein are exemplary in nature, and that these specific embodiments or examples are not to be considered in a limiting sense, because numerous variations are possible. The specific routines or methods described herein may represent one or more of any number of processing strategies. As such, various acts illustrated and/or described may be performed in the sequence illustrated and/or described, in other sequences, in parallel, or omitted. Likewise, the order of the above-described processes may be changed.

The subject matter of the present disclosure includes all novel and non-obvious combinations and sub-combinations of the various processes, systems and configurations, and other features, functions, acts, and/or properties disclosed herein, as well as any and all equivalents thereof.

## Claims

1. A method (400) of forming a vector database using a fine-tuned neural network, the method comprising:
inputting (402) labeled training data into a pre-trained neural network configured to form embeddings of chemical structures, the labeled training data comprising structural information and a value of a property for each chemical object in a first set of chemical objects;
fine-tuning (408) the pre-trained neural network using the labeled training data, thereby forming the fine-tuned neural network; and
forming (414) the vector database by
inputting a reference dataset into the fine-tuned neural network to generate embeddings of chemical structures, the reference dataset comprising structural information for each chemical object in a second set of chemical objects, each embedding comprising a vector representation of a chemical structure and embedded information for the property for the chemical structure, and
storing the embeddings with metadata in the vector database.

2. The method of claim 1, further comprising,
prior to inputting the labeled training data, inputting a set of pre-training data into a neural network, the set of pre-training data comprising structural information for each chemical object of a third set of chemical objects; and
training the neural network to form the pre-trained neural network.

3. The method of either of claims 1 or 2, further comprising:
receiving a query comprising chemical structure information for a target chemical object;
based on the query, inputting the chemical structure information for the target chemical object into the fine-tuned neural network;
receiving an embedding for the target chemical object from the fine-tuned neural network, the embedding comprising a vector representation of the target chemical object;
based at least on a similarity score between the embedding for the target chemical object and each of one or more embeddings stored in the vector database, retrieving query results from the vector database, the query results comprising a set of embeddings and metadata for a corresponding set of chemical objects; and
outputting the query results.

4. The method of claim 3, wherein outputting the query results comprises, for each chemical object in the corresponding set of chemical objects of the query results, outputting structural information and a predicted property value for the chemical object.

5. The method of either of claim 3 or 4, wherein retrieving the query results from the vector database comprises determining one or more of a Euclidean distance, a Manhattan distance, a dot product, or a cosine similarity for the embedding for the target chemical object and an embedding stored in the vector database.

6. The method of any of claims 1 to 5, wherein the value for the property comprises one or more of a scalar value or a classification for the property for each chemical object in the first set of chemical objects.

7. The method of any of claims 1 to 6, wherein the labeled training data comprises values for two of more properties for each chemical object in the first set of chemical objects.

8. The method of any of claims 1 to 7, wherein fine-tuning the neural network comprises fine-tuning a graph neural network (GNN).

9. A method (400) enacted on a computing system, the method comprising:
inputting (402) labeled training data into a pre-trained neural network configured to form embeddings of chemical structures, the labeled training data comprising structural information and a value for a property for each chemical object in a set of chemical objects;
fine-tuning (408) the pre-trained neural network using the labeled training data, thereby forming a fine-tuned neural network;
forming (414) a vector database by using the fine-tuned neural network to generate embeddings of chemical structures of a reference dataset, each embedding comprising a vector representation of a chemical structure and embedded information for the property for the chemical structure, and storing the embeddings with metadata in the vector database;
receiving (418) a query comprising chemical structure information for a target chemical object;
based on the query, inputting (418) the chemical structure information into the fine-tuned neural network;
receiving (418) an embedding for the target chemical object from the fine-tuned neural network, the embedding for the target chemical object comprising a vector representation of the target chemical object;
based at least on a similarity score between the embedding for the target chemical object and each of one or more embeddings stored in the vector database, retrieving (418) query results from the vector database, the query results comprising a set of embeddings and metadata for a corresponding set of chemical objects; and
outputting (418) the query results.

10. The method of claim 9, further comprising,
prior to inputting the labeled training data, inputting a set of pre-training data into a neural network, the set of pre-training data comprising structural information for each chemical object of the dataset; and
training the neural network to form the pre-trained neural network.

11. A computing system (104, 500) implementing a neural network configured to produce embeddings of chemical structures, the computing system comprising:
a logic subsystem (502); and
a storage subsystem (504) comprising instructions executable by the logic subsystem to
input (402) labeled training data into a pre-trained neural network configured to form embeddings of chemical structures, the labeled training data comprising structural information and a value for a property for each chemical object in a first set of chemical objects,
train (408) the neural network to predict the property, thereby forming a fine-tuned neural network,
input a reference dataset into the fine-tuned neural network to generate embeddings of chemical structures, the reference dataset comprising structural information for a second set of chemical objects, each embedding comprising a vector representation of a chemical structure and embedded information for the property for the chemical structure, and
store (414) the embeddings with metadata in a vector database.

12. The computing system of claim 11, wherein the instructions are further executable to:
receive a query comprising chemical structure information for a target chemical object,
based on the query, input the chemical structure information into the fine-tuned neural network,
receive, from the fine-tuned neural network, an embedding comprising a vector representation of the target chemical object,
based at least on a similarity score between the embedding for the target chemical object and each of one or more embeddings stored in the vector database, retrieve query results from the vector database, the query results comprising a set of embeddings and metadata for a corresponding set of chemical objects, and
output the query results.

13. The computing system of claim 12, wherein the instructions executable to output the query results are executable to output structural information and predicted property values for each chemical object in the corresponding set of chemical objects.

14. The computing system of any of claims 11 to 1813 wherein the labeled training data comprises values for two or more properties for each chemical object in the first set of chemical objects, and the instructions are executable to train the neural network to predict the two or more properties.

15. The computing system of any of claims 11 to 14, wherein the instructions executable to train the neural network to predict the property comprise instructions executable to train a graph neural network to predict one or more of a boiling point, a flash point, an electronegativity, an ionization energy, a bandgap, or a dielectric constant.
